(19) Europäisches Patentamt
European Patent Office
Office européen des brevets

(11) **EP 4 740 991 A2**

(12) **EUROPÄISCHE PATENTANMELDUNG**

(43) Veröffentlichungstag:
**13.05.2026   Patentblatt 2026/20**

(21) Anmeldenummer: 26169543.1

(22) Anmeldetag: **11.09.2019**

(51) Internationale Patentklassifikation (IPC):
***A61M 25/10*** *(2013.01)*

(52) Gemeinsame Patentklassifikation (CPC):
**A61L 29/16; A61M 25/1029;** A61L 2420/02;
A61M 2025/1031

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **19.09.2018   DE 102018123050**

(62) Dokumentnummer(n) der früheren Anmeldung(en)
nach Art. 76 EPÜ:
**19782478.2 / 3 852 825**

(71) Anmelder: **Ruebben, Alexander
98000 Monaco (MC)**

(72) Erfinder: **Ruebben, Alexander
98000 Monaco (MC)**

(74) Vertreter: **Schneiders & Behrendt Bochum
Gerard-Mortier-Platz 6
44793 Bochum (DE)**

Bemerkungen:
Diese Anmeldung ist am 31.03.2026 als
Teilanmeldung zu der unter INID-Code 62 erwähnten
Anmeldung eingereicht worden.

(54) **WIRKSTOFFBESCHICHTUNG FÜR BALLONS VON BALLONKATHETERN**

(57)   Die Erfindung betrifft ein Verfahren zur Beschichtung der Oberfläche des Ballons eines Ballonkatheters mit einem Wirkstoff, wobei der Ballon aus einem elastischen Material gefertigt und durch Druckbeaufschlagung mit einem Fluid expandierbar ist, wobei der Ballon dafür vorgesehen ist, an einem Zielort expandiert zu werden, wobei die Beschichtung der Oberfläche des Ballons bei einem Druck erfolgt, der unterhalb des Drucks liegt, der für die Expansion des Ballons am Zielort aufgewandt wird. Auf diese Weise wird aufgrund des Entstehens von Scherkräften eine besonders effektive Abgabe des Wirkstoffs vom Ballon auf die Blutgefäßinnenwandung bzw. das umliegende Gewebe erreicht. Des Weiteren betrifft die Erfindung einen entsprechenden Ballon sowie einen Ballonkatheter.

EP 4 740 991 A2

**Beschreibung**

Die Erfindung betrifft ein Verfahren zur Beschichtung der Oberfläche des Ballons eines Ballonkatheters mit einem Wirkstoff, wobei der Ballon aus einem elastischen Material gefertigt und durch Druckbeaufschlagung mit einem Fluid expandierbar ist, wobei der Ballon dafür vorgesehen ist, an einem Zielort expandiert zu werden.

Die sogenannten "minimalinvasiven Verfahren" nehmen in der Medizin einen immer größeren Stellenwert ein. Zur Behandlung von Gefäßverengungen wie Arteriosklerose wird häufig die perkutane transluminale Angioplastie (PTA) mittels Ballondilatation eingesetzt. Hierbei wird ein Ballonkatheter, der im distalen Bereich einen durch Fluidzufuhr expandierbaren Ballon aufweist, mit Hilfe eines Führungskatheters zur Stenose (Gefäßverengung) gebracht. Dort erfolgt eine Aufweitung des Ballons, wodurch den Blutfluss hemmende Ablagerungen an oder in die Gefäßwand gedrückt werden, so dass ein ungestörter Blutfluss wieder möglich ist. Nach erfolgter Behandlung und dem anschließenden Zusammenfalten des Ballons wird der Ballonkatheter aus dem Gefäßsystem zurückgezogen und entfernt.

In manchen Fällen kann es in der Folge einer zunächst erfolgreich durchgeführten Angioplastie zu einer erneuten Verengung in dem behandelten Gefäßabschnitt kommen. Solch eine Restenose ist meist auf eine Zellproliferation im entsprechenden Gefäßabschnitt zurückzuführen, d. h. Zellen des Blutgefäßes wachsen in das Gefäßlumen hinein und sorgen wiederum für eine Behinderung des Blutflusses. Um dies zu verhindern, werden zunehmend mit Medikamenten beschichtete Ballonkatheter eingesetzt. Entsprechende Medikamente wirken meist proliferationshemmend insbesondere auf die *Smooth Muscle Cells* (SMC) und sollen so eine durch ein übermäßiges Wachstum dieser Zellen bedingte Restenose verhindern. Das Medikament befindet sich auf der Außenseite des Ballons und wird während der Ballondilatation vom Ballon auf beziehungsweise in die Gefäßinnenwand übertragen.

Typischerweise wird der Ballon des Ballonkatheters beschichtet, indem ein in einem Lösungsmittel gelöster Wirkstoff auf die Oberfläche des Ballons aufgebracht wird, wobei das Lösungsmittel nach Aufbringung der Lösung verdunstet. Der Wirkstoff befindet sich dann als Schicht auf der Oberfläche und kann während der Ballondilatation appliziert werden.

Als problematisch hat sich die Übertragung des Wirkstoffs von der Oberfläche des Ballons auf die Gefäßinnenwand herausgestellt. Dabei ist zu berücksichtigen, dass die Expansion des Ballons zeitlich eng begrenzt sein muss, weil ein Verschluss des Blutgefäßes über zu lange Zeiträume eine Ischämie und eine Unterversorgung des Gewebes oder von Organen bis hin zum Infarkt bewirken würde. Entsprechend muss die Wirkstoffübertragung innerhalb eines vergleichsweise kurzen Zeitraums erfolgen. Im koronaren Bereich kann der Ballon max. für einen Zeitraum von 30 bis 60 s expandiert werden. Bekannte medikamentenbeschichtete Ballone benötigen für eine ausreichende Wirkstoffabgabe jedoch häufig einen längeren Zeitraum. Dies führt entweder zu den genannten ischämischen Problemen oder zu einer unzureichenden Wirkstoffabgabe durch eine notwendige zeitliche Verkürzung der Ballonexpansion.

Des Weiteren muss sichergestellt sein, dass sich der Wirkstoff erst am Zielort von der Ballonoberfläche löst, zumal es sich bei den verwendeten Wirkstoffen häufig um toxische Substanzen wie Paclitaxel handelt, bei denen die Freisetzung abseits vom Zielort unerwünscht ist. Zudem muss die Sicherheit für das behandelnde medizinische Personal gewährleistet sein. Es besteht somit ein Zielkonflikt, einerseits muss der Wirkstoff bzw. das Medikament am Zielort möglichst schnell abgegeben und auf die Innenwandung des Blutgefäßes übertragen werden, andererseits soll aber der Wirkstoff bei der Vorbereitung und beim Vorschub des Ballonkatheters möglichst fest auf dem Ballon haften. In der Regel erfolgt jedoch die Abgabe eines stark haftenden Wirkstoffs nur langsam.

Es stellt sich somit die Aufgabe, einen Ballon zur Verfügung zu stellen, der einerseits die Wirkstoffbeschichtung sicher hält, andererseits jedoch bei Expansion am Zielort den Wirkstoff rasch abgibt.

Diese Aufgabe wird erfindungsgemäß gelöst durch ein Verfahren zur Beschichtung der Oberfläche des Ballons eines Ballonkatheters mit einem Wirkstoff, wobei der Ballon aus einem elastischen Material gefertigt und durch Druckbeaufschlagung mit einem Fluid expandierbar ist, wobei der Ballon dafür vorgesehen ist, an einem Zielort expandiert zu werden, wobei die Beschichtung der Oberfläche des Ballons bei einem Druck erfolgt, der unterhalb des Drucks liegt, der für die Expansion des Ballons am Zielort aufgewandt wird.

Bei Ballons für Ballonkatheter muss grundsätzlich unterschieden werden zwischen non-compliant, semi-compliant und compliant Ballons. Der Unterschied zwischen den Ballons liegt in der unterschiedlichen Zunahme des Durchmessers bei Befüllung des Ballons mit einem Fluid unter einem bestimmten Druck. Als Compliance wird definiert:

Compliance (in %) = (d (hoher Druck) - d (niedriger Druck))/d (niedriger Druck) $\times$ 100 %, wobei d der Durchmesser des Ballons ist.

Als hoher bzw. niedriger Druck können dabei die Endpunkte der sog. Working Range angesehen werden, wobei sich die Working Range zwischen dem Nominaldruck, bei dem der Ballon seinen nominalen Durchmesser erreicht, und dem Maximaldruck erstreckt, auf den der Ballon ohne Beschädigung gebracht werden kann. Je nach prozentualer Durchmesserzunahme werden dabei

non-compliant Ballons, Durchmesserzunahme (Compliance): 0 bis 7 %,

semi-compliant Ballons, Durchmesserzunahme (Compliance): 5 bis 10 % und

compliant Ballons, Durchmesserzunahme (Compliance): 10 bis 500 % unterschieden.

In der Literatur lassen sich zum Teil unterschiedliche Definitionen für die Abgrenzung zwischen non-compliant, semi-compliant und compliant finden, grundsätzlich gilt aber, dass der Durchmesser eines compliant Ballons mit dem Druck deutlich zunimmt, während der Durchmesser eines non-compliant Ballons auch bei hohem Druck nahezu konstant bleibt. Non-compliant Ballons sind aus einem weitgehend unelastischen Material gefertigt. Semi-compliant Ballons liegen hinsichtlich der Durchmesserzunahme zwischen non-compliant und compliant Ballons.

Für sämtliche Ballons existieren sinnvolle Anwendungsmöglichkeiten. Darüber hinaus weisen alle Ballons bestimmte Vor- und Nachteile auf. Compliant Ballons sind flexibler, lassen sich daher leichter einführen und eignen sich für Anwendungen, bei denen eine Anpassung an die Form des Gefäßes gewünscht ist, bei weit fortgeschrittenen Gefäßverengungen und zur Prä-Dilatation vor dem Setzen eines Stents. Ein non-compliant Ballon hingegen eignet sich, wenn der Ballon über die gesamte Länge auf einen bestimmten Durchmesser gebracht werden soll, beispielsweise um sehr feste, verkalkte Gefäßwandablagerungen nach außen zu drücken oder einen bereits platzierten Stent fest und gleichmäßig in Richtung Gefäßwand zu pressen (Post-Dilatation). Beim Pressen gegen Gefäßwandablagerungen zeigt ein non-compliant Ballon eine im Wesentlichen zylindrische Struktur, während sich ein semi-compliant oder compliant Ballon sich bei Ablagerungen, die sich nur über einen kurzen Abschnitt erstrecken, proximal und distal der Ablagerung stärker ausdehnt als im Bereich der Ablagerung selbst. In diesem Zusammenhang wird auch vom dog-bone-Effekt gesprochen.

Da die Ballonoberfläche A jedenfalls im bei expandiertem Ballon zylindrischen Bereich über die Beziehung

$$A = \pi \times d \times L$$

mit dem Durchmesser d und der Länge L in Zusammenhang steht, nimmt die Oberfläche, eine konstante Länge des Ballons unterstellt, in gleichem Maße wie der Durchmesser zu, d. h. die oben wiedergegebenen Definitionen zur Compliance gelten in gleicher Weise für die Ballonoberfläche. Soweit im Rahmen dieser Erfindung vom Durchmesser des Ballons gesprochen wird, ist hierunter stets der Außendurchmesser zu verstehen.

Die Erfindung bezieht sich auf semi-compliant oder compliant Ballons, d. h. Ballons, die aus einem Material gefertigt sind, das eine gewisse Elastizität aufweist. Ein solcher Ballon wird erfindungsgemäß bei einer geringen Druckbeaufschlagung mit einem Wirkstoff beschichtet, während der Druck, mit dem der Ballon am Zielort expandiert wird, höher liegt. Bei einem Ballon mit einer gewissen Compliance führt dies dazu, dass sich der Durchmesser und damit auch die Ballonoberfläche vergrößert. Da der Druck am Zielort, an dem der auf die Ballonoberfläche aufgebrachte Wirkstoff an die Gefäßinnenwandung abgegeben werden soll, höher ist als der Druck, unter dem der Ballon mit Wirkstoff beschichtet wurde, kommt es innerhalb der Wirkstoffschicht zu Scherkräften. Der Wirkstoff wird daher vom Ballon jedenfalls teilweise abgesprengt und löst sich vom Ballon. Auf diese Weise können auch stark anhaftende Beschichtungen abgelöst werden.

Die erfindungsgemäßen Ballonkatheter können in Blutgefäßen eingesetzt werden, insbesondere im Bereich der Angioplastie. In diesem Fall ist der Zielort des Ballons ein Blutgefäß. Es ist jedoch auch möglich, Ballonkatheter in anderen medizinischen Bereichen einzusetzen. Eine Einsatzmöglichkeit liegt in der Urologie, wo Ballonkatheter als Blasenkatheter in die Harnblase eingesetzt werden. Über den Ballon wird der Katheter fixiert. Hier kann der Ballon z. B. mit einer Beschichtung versehen sein, die bakterieller Besiedlung und Inkrustation vorbeugt, beispielsweise mit Heparin.

In der Pneumologie können Ballonkatheter zur Aufweitung oder zum Verschluss eines Bronchus eingesetzt werden. Auch in der Gynäkologie können Ballonkatheter zum Einsatz kommen. Im Bereich der Orthopädie können Ballonkatheter zur Behandlung von Wirbelbrüchen durch Wiederaufrichtung der Wirbel mittels Ballonexpansion eingesetzt werden (Ballon-Kyphoplastie). Der erfindungsgemäße Ballonkatheter ist grundsätzlich auf sämtlichen Gebieten der Medizin einsetzbar, in denen beschichtete Ballonkatheter zum Einsatz kommen.

Vorzugsweise werden erfindungsgemäß Ballons eingesetzt, bei denen die Durchmesserzunahme bei Verdopplung des Drucks, ausgehend vom Nominaldruck, bei dem der Ballon seinen normalen bzw. nominalen Durchmesser erreicht, mindestens 5 % beträgt, vorzugsweise mindestens 10 %, weiter vorzugsweise mindestens 20 % und besonders bevorzugt mindestens 30 %.

Die Erfindung weist den zusätzlichen Vorteil auf, dass die Ablösung der Beschichtung erst bei Expansion des Ballons erfolgt, d. h. erst am Zielort, an dem tatsächlich der Wirkstoff beispielsweise auf die Blutgefäßinnenwandung übertragen werden soll. Im Gegensatz dazu erfolgt im komprimierten Zustand des Ballons keine Ablösung von Wirkstoff; mit anderen Worten wird kein Wirkstoff in nicht hierfür vorgesehenen Bereichen des Blutgefäßsystems freigesetzt. Ebenso erfolgt keine Freisetzung oder Ablösung von Wirkstoff außerhalb des Körpers, die mit dem Ballonkatheter in Kontakt kommende Personen gefährden könnte.

Die Expansion des Ballons wird somit bewusst ausgenutzt, um Wirkstoff vom Ballon insbesondere auf eine Blutgefäßinnenwandung zu übertragen. Um eine gleichmäßige Beschichtung des Ballons zu erreichen, sollte der Druck, der auf den Ballon einwirkt und bei dem die Beschichtung durchgeführt wird, so hoch sein, dass sich der Ballon komplett oder zumindest weitgehend entfaltet, jedoch niedriger als der Druck, mit dem der Ballon typischerweise am Zielort im Blutgefäß beaufschlagt wird. Beispielsweise kann ein Ballon bei einer Druckbeaufschlagung von 3 bar beschichtet werden, während die Druckbeaufschlagung im Blutgefäß 6 bar beträgt. Der Durchmesser eines compliant Ballons kann dabei z. B. von 4,5 mm auf 6 mm zunehmen, dies bedeutet eine Zunahme des Durchmessers und damit auch der Ballonoberfläche um 33 %. Diese bewirkt eine starke, die Absprengung des Wirkstoffes herbeiführende Scherkraft.

Vorteilhafterweise liegt der Druck, bei dem die Beschichtung erfolgt, mindestens 20 %, weiter bevorzugt mindestens 30 % unterhalb des Drucks, der für die Expansion des Ballons am Zielort aufgewandt wird. Weiter vorteilhafterweise beträgt der Druck, bei dem die Beschichtung erfolgt, max. 50 % des Drucks, der für die Expansion des Ballons am Zielort aufgewandt wird. Die Zunahme der Ballonoberfläche zwischen dem Beschichtungsvorgang und dem Expansionsvorgang am Zielort sollte mindestens 10 %, vorteilhafterweise mindestens 20 %, weiter vorteilhafterweise mindestens 30 %, weiter vorteilhafterweise mindestens 40 % und weiter vorteilhafterweise mindestens 50 % betragen.

Der Ballon ist, um die der Erfindung zugrunde liegende Expansionsfähigkeit zu gewährleisten, mindestens teilweise aus einem elastischen Material gefertigt. Als elastisches Material kann z. B. ein Polyurethan, ein Polyolefincopolymer, ein Polyethylen oder ein Silikon verwendet werden. Weitere verwendbare Materialien sind thermoplastische Elastomere, insbesondere Polyetherblockamide (PEBA). Hierbei handelt es sich um ein thermoplastisches Elastomer, das durch Polykondensation eines Carbonsäurepolyamids mit einem Polyether mit endständigen OH-Gruppen erhältlich ist. Insbesondere wird PEBA unter der Bezeichnung PEBAX® von der Firma Arkema vertrieben. Auch Polyamide wie Nylon (Polyhexamethylenadipinsäureamid) mit einer gewissen Elastizität lassen sich zumindest für semi-compliant Ballons verwenden.

Alternativ können auch andere Polyamide als elastisches Material für den Ballon verwendet werden, beispielsweise solche, wie sie unter der Bezeichnung Grilamid® von der Firma EMS-GRIVORY vertrieben werden. Besonders bevorzugt ist die Verwendung eines Polyamids 12 (PA 12, Grilamid® L), einem durch die Polykondensation von Laurinlactam erhältlichen Polyamid. Weitere verwendbare Polyamide sind Polyamid 10.10 (PA 10.10, Grilamid® 1S), ein durch Polykondensation von Decandiamin und Sebacinsäure erhältliches Polyamid, Polyamid 6.10 (PA 6.10, Grilamid® 2S), ein durch Polykondensation von Hexamethylendiamin und Sebacinsäure erhältliches Polyamid oder Polyamid 6.12 (PA 6.12, Grilamid® 2D), ein durch Polykondensation von Hexamethylendiamin und Dodecandisäure erhältliches Polyamid.

Bei dem verwendeten Wirkstoff handelt es sich insbesondere um ein Arzneimittel bzw. Medikament, bevorzugt um ein Arzneimittel, das proliferationshemmend wirkt und das gefäßverengende Überwuchern der durch den Ballon erweiterten Stelle verhindert. Ebenso kann es sich um einen hormonartigen oder regulierenden Wirkstoff handeln, der an bestimmten Zellen organspezifische Wirkungen oder Regulationsfunktionen beeinflussen kann. Insbesondere kann der Wirkstoff ausgewählt sein aus: Tretinoin, Orphanrezeptoragonisten, Elafinderivate, Corticosteroide, Steroidhormone, Paclitaxel, Rapamycin, Tacrolimus, hydrophobe Proteine sowie zellproliferationsverändernde Substanzen. Es ist auch möglich, Gemische dieser Wirkstoffe zu verwenden. Darüber hinaus können auch Derivate der genannten Wirkstoffe verwendbar sein, wobei unter Derivaten insbesondere Salze, Ester und Amide verstanden werden. Als Steroidhormone können beispielsweise Methylprednisolon, Dexamethason oder Östradiol verwendet werden. Besonders bevorzugt ist die Verwendung von Paclitaxel, Rapamycin oder Tacrolimus bzw. entsprechenden Derivaten.

Allgemein ist der Begriff Wirkstoff jedoch weit zu verstehen, d. h. es kann sich grundsätzlich um beliebige Beschichtungen auf dem Ballon des Ballonkatheters handeln, mit denen am Zielort eine bestimmte Wirkung erzielt werden soll. Diese Wirkung kann bei Einbringung in Blutgefäße insbesondere in der Zellproliferationshemmung bestehen. Auf anderen Gebieten der Medizin kann die gewünschte Wirkung jedoch eine andere sein, so etwa im Bereich der Urologie bei Blasenkathetern, wo die Beschichtung insbesondere der Hemmung der Bakterienbesiedlung dienen soll. Hier kann als Wirkstoff beispielsweise Heparin eingesetzt werden.

Die Beschichtung der Oberfläche des Ballons mit dem Wirkstoff erfolgt typischerweise dadurch, dass die Oberfläche des Ballons mit einer Lösung des Wirkstoffs in Kontakt gebracht wird. Dies kann insbesondere durch Eintauchen des Ballons in die Lösung erfolgen. Das Eintauchen dauert dabei in der Regel max. 1 min, typischerweise 10 bis 30 s. Der Ballon sollte nach dem Eintauchen mit einer Geschwindigkeit von bis zu 10 mm/s aus der ersten Lösung herausgezogen werden. Noch günstiger ist es, wenn das Herausziehen mit einer Geschwindigkeit von weniger als 5 mm/s, vorzugsweise mit einer Geschwindigkeit zwischen 0,5 mm/s und 2 mm/s erfolgt. Durch das langsame Herausziehen wird ein langsames Trocknen der Oberfläche erreicht.

Vor der Beschichtung des Ballons ist es sinnvoll, die Oberfläche des Ballons zu reinigen. Dies kann beispielsweise mit einem entsprechenden Lösungsmittel erfolgen, beispielsweise dem auch für die Aufbringung des Wirkstoffs verwendeten Lösungsmittel.

Die Lösung kann hinsichtlich des Wirkstoffs gesättigt sein, dies ist jedoch nicht unbedingt erforderlich. Als Lösungsmittel können beispielsweise Methylenchlorid, Chloroform, Alkohol, insbesondere Ethanol, Methanol oder Isopropanol, Aceton, Diethylether, flüssige Kohlenwasserstoffe, wie zum Beispiel Pentan, Hexan, Heptan, Cyclohexan oder Octan, Toluol, Tetrahydrofuran (THF) oder Essigester verwendet werden. Möglich ist auch die Verwendung von Lösungsmittelgemischen. Vorzugsweise handelt es sich um eine Lösung des Wirkstoffs in Methylenchlorid.

Alternativ zur Beschichtung durch Eintauchen kann diese auch auf andere Weise erfolgen, bspw. durch Besprühen.

Beim Einsatz des erfindungsgemäßen Ballonkatheters wird dieser in das Blutgefäßsystem bzw. ein anderes Körperlumen eingeführt und durch Aufblasen an die Innenwand des Gefäßes/Lumens angepresst. Dabei wird ein Großteil der Beschichtung auf die Innenwand übertragen. Nach Ablassen des Drucks und Entfernung des Ballonkatheters aus dem Gefäßsystem/Lumen dringt der innerhalb der Beschichtung aufgebrachte Wirkstoff nach und nach in das Gewebe ein.

Unter Ballon im Sinne der Erfindung wird das durch Zufuhr eines Fluids expandierbare Element eines Ballonkatheters verstanden, unabhängig davon, welche Form das expandierbare Element aufweist oder aus welchem Material es besteht. Das Fluid kann gasförmig oder flüssig sein. Bevorzugt ist ein Gas, bspw. Luft. Der Druck, mit dem der Ballon zur Expansion im Blutgefäß/Lumen beaufschlagt wird, liegt typischerweise zwischen 5 und 15 bar. Die Dimensionen des Ballons können je nach Anwendungsbereich stark differieren, der Durchmesser im expandierten Zustand kann beispielsweise zwischen ca. 1 und ca. 50 mm liegen, die Länge zwischen ca. 5 und ca. 300 mm. Ggf. können die Maße hiervon aber auch abweichen, beispielsweise bei Einsatz des Ballons/Ballonkatheters in der Urologie oder Veterinärmedizin.

Ballonkatheter sind grundsätzlich hinlänglich im Stand der Technik bekannt und weisen einen langgestreckten, von proximal nach distal verlaufenden Katheter sowie einen im distalen Bereich angeordneten Ballon auf. Es handelt sich um einen Katheter, der hinsichtlich seiner Dimensionen auf die Einführung in ein Körperlumen, insbesondere ein (Blut)gefäßsystem, abgestimmt ist. Dabei können die exakten Dimensionen variieren, je nachdem, ob das Blutgefäß bspw. eine Koronararterie, ein intrakranielles Blutgefäß oder eine Unterschenkelarterie ist. Darüber hinaus verfügt der Ballonkatheter über Mittel zur Zuführung eines Fluids zum Ballon. Hierbei kann es sich um ein Zufuhrlumen handeln, das sich über die Länge des Ballonkatheters erstreckt.

Darüber hinaus kann der erfindungsgemäße Ballonkatheter nicht nur der Beseitigung von Stenosen und der lokalen Wirkstoffeinbringung, sondern zusätzlich der Platzierung eines Stents (Endoprothese) im Körperlumen dienen. Stents sind röhrenartige Stützstrukturen, die in einem Körperlumen, bspw. einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Körperlumen eingebracht. An der vorgesehenen Stelle wird sodann der Ballon durch Zufuhr eines Fluids expandiert, wodurch sich auch der Stent weitet und im Körperlumen verankert wird. Gleichzeitig wird bei Verwendung des erfindungsgemäßen Ballons der Wirkstoff an die Wandung des Körperlumens abgegeben. Schließlich wird der Ballon wieder zusammengezogen und aus dem Körperlumen entfernt, während der Stent im Körperlumen verbleibt.

Gemäß einer bevorzugten Ausführungsform wird zumindest der mit dem Wirkstoff beschichtete Teil der Oberfläche des Ballons mit einer Wasser und/oder mindestens einen Alkohol enthaltenden Flüssigkeit benetzt. Bei der Beschichtung der Oberfläche des Ballons mit einem Wirkstoff wird auf der Oberfläche zumeist eine lackartige, transparente Wirkstoffschicht erzeugt, die als Basis für eine homogene und reproduzierbare Wirkstoffbeladung dient. Diese Beschichtung wird durch die Wasser und/oder mindestens einen Alkohol enthaltende Flüssigkeit angegriffen und die Oberfläche wird poröser beziehungsweise versprödet partiell. Die gesamte Beschichtung wird spröder und optisch weniger transparent, also milchiger. Die so erzeugte Oberfläche hat eine kreideartige, unter Umständen auch nichtkristalline Konsistenz, die einen höheren Wirkstoffabtrag bei Reibung ermöglicht als im Falle einer Beschichtung nur durch Benetzen der Oberfläche des Ballons mit einer Lösung des Wirkstoffs. Ein entsprechendes Verfahren ist grundsätzlich aus der WO 2013/178820 A1 bekannt.

Bei der Wasser und/oder mindestens einen Alkohol enthaltenden Flüssigkeit handelt es sich insbesondere um eine einen Alkohol und/oder ein Keton enthaltende wässrige Lösung. Die Konzentration des Alkohols und/oder Ketons in der wässrigen Lösung beträgt typischerweise 10 bis 70 % (v/v), bevorzugt 30 bis 65 % (v/v), weiter bevorzugt 50 bis 60 % (v/v) und ganz besonders bevorzugt ca. 55 % (v/v). Verwendbar sind grundsätzlich mit Wasser mischbare Alkohole und Ketone, wobei auch eine Mischung aus mehreren Alkoholen und/oder Ketonen verwendet werden kann, für die dann die oben genannten, bevorzugten Konzentrationsangaben insgesamt gelten. Bevorzugt ist die Verwendung von Ethanol, Methanol, Aceton und/oder Isopropanol. Am meisten bevorzugt ist Ethanol. Weiterhin kann die wässrige Lösung ein azeotropes Lösungsmittelgemisch umfassen, insbesondere ein Alkohol/Wasser-Gemisch, bevorzugt ein Ethanol/Wasser-Gemisch. Denkbar wäre auch, in der Wasser und/oder mindestens einen Alkohol enthaltenden Flüssigkeit eine zusätzliche Menge Wirkstoff vorzusehen, um die Beladung des Ballons mit Wirkstoff zu erhöhen.

Gemäß einer weiteren vorteilhaften Ausführungsform der Erfindung wird zumindest der mit dem Wirkstoff beschichtete Teil der Oberfläche des Ballons vor oder nach der Beschichtung mit dem Wirkstoff mit einem Polysaccharid beschichtet. Es ist auch möglich, zunächst eine Beschichtung mit Wirkstoff, anschließend eine Beschichtung mit einem Polysaccharid und schließlich erneut mit Wirkstoff vorzunehmen. Überraschend hat sich herausgestellt, dass die Polysaccharidbeschichtung ähnlich einem Klebstoff auf der Innenwand des behandelten Gefäßes wirkt, d. h. der Wirkstoff haftet erheblich besser auf der Gefäßwand und wird weniger leicht vom Blutstrom mitgerissen. Entsprechend kann der Wirkstoff über einen langen Zeitraum seine Wirkung entfalten und aus der Polysaccharidbeschichtung nach und nach in das Gewebe des Gefäßes gelangen. Es konnte gezeigt werden, dass selbst nach einigen Wochen noch signifikante Wirkstoffkonzentrationen nachweisbar sind.

Polysaccharide stellen eine hydrophile Beschichtung dar, die in einer wässrigen Umgebung wie Blut eine gewisse Quellung bzw. Aufweichung erfährt. Dies führt dazu, dass der Wirkstoff während der Ballondilatation gut auf die Innenwand des Gefäßes übertragen wird. Das erfindungsgemäße Verfahren eignet sich insbesondere zum Aufbringen von lipophilen Beschichtungen auf den Ballon. Es hat sich nämlich herausgestellt, dass gerade die hydrophilen Polysaccharide gut geeignet sind, dafür zu sorgen, dass lipophile Wirkstoffe während der Ballondilatation effektiv auf die Innenwände der behandelten Gefäße übertragen werden und eine langanhaltende Wirkstoffkonzentration bewirken.

Auch bei der Beschichtung mit dem Polysaccharid liegt dieses bevorzugt in einer Lösung vor, bevorzugt in einer alkoholischen Lösung. Diese kann neben einem oder mehreren Alkoholen insbesondere auch Wasser enthalten. Eine wässrigalkoholische Lösung ist insofern von Vorteil, als sie das Polysaccharid gut löst, eine bereits zuvor aufgebrachte Wirkstoffschicht jedoch nicht wieder abträgt. Darüber hinaus sorgt der organische Anteil in der Lösung für eine rasche Trocknung nach der Benetzung. Die Konzentration des Alkohols bzw. der Alkohole in der weiteren Lösung beträgt typischerweise 10 bis 70 % (v/v), bevorzugt 30 bis 65 % (v/v), weiter bevorzugt 50 bis 60 % (v/v) und besonders bevorzugt ca. 55 % (v/v). Als Alkohol verwendbar sind solche Alkohole, die das Polysaccharid lösen. In der Regel sind derartige Alkohole auch mit Wasser mischbar. Bevorzugt sind Ethanol, Methanol und Isopropanol, besonders bevorzugt ist Ethanol.

Die mittlere Molmasse des Polysaccharids beträgt zweckmäßigerweise 10.000 bis 100.000.000 Da. Als besonders zweckmäßig hat sich eine mittlere Molmasse zwischen 20.000 und 80.000 Da herausgestellt. Der Polysaccharid-Gehalt der weiteren Lösung beträgt vorzugsweise 1 bis 15 Gew.-%, weiter bevorzugt 2 bis 10 Gew.-% und besonders bevorzugt 3 bis 8 Gew.-%.

Bei dem Polysaccharid handelt es sich bevorzugt um ein verzweigtes Polysaccharid. Geeignet sind auch Gemische aus mehreren Polysacchariden und modifizierte Polysaccharide. Bevorzugt sind Dextrane, insbesondere natürliche Dextrane. Bei Dextranen handelt es sich um hochmolekulare, verzweigte Polymere, die sich aus Glucoseeinheiten zusammensetzen. Sie werden u. a. von Bakterien der Gattung *Leuconostoc* hergestellt. Verwendung finden sie als Blutplasma-Ersatzmittel oder als Träger in der Chromatographie.

Bei dem Dextran kann es sich insbesondere um ein natürliches Dextran handeln. Besonders bevorzugt ist Dextran 40 mit einer mittleren Molmasse von ca. 40.000 Da. Neben Dextranen können jedoch grundsätzlich auch andere Polysaccharide Verwendung finden. Ein Beispiel für ein verwendbares modifiziertes Polysaccharid ist Hydroxyethylstärke (HES).

Grundsätzlich kann sowohl die gesamte Ballonoberfläche oder nur ein Teil der Ballonoberfläche, beispielsweise der beim Aufdehnen mit dem Gewebe in Kontakt kommende Bereich der Oberfläche, mit dem erfindungsgemäßen Verfahren beschichtet werden. Insbesondere kann der Ballon einen zylinderförmigen Bereich und mindestens einen konusförmigen Bereich umfassen. In diesem Fall kann beispielsweise nur der zylinderförmige Bereich des Ballons mit einem Wirkstoff erfindungsgemäß beschichtet werden oder der zylinderförmige Bereich des Ballons und ein konusförmiger Bereich.

Auch die Benetzungen bzw. Beschichtungen der Oberfläche des Ballons mit einer Wasser und/oder mindestens einen Alkohol enthaltenden Flüssigkeit oder der ein Polysaccharid enthaltenden Flüssigkeit können durch Eintauchen des Ballons in die Flüssigkeit erfolgen, ähnlich wie die zuvor beschriebene Beschichtung mit dem Wirkstoff. Es ist sinnvoll, auch diese Benetzungen bei dem Druck durchzuführen, bei dem auch die Beschichtung mit dem Wirkstoff erfolgt, um ein gleichmäßiges Ablösen der Beschichtung bei stärkerer Expansion des Ballons am Zielort im Blutgefäß zu gewährleisten. Als identischer Druck wird in diesem Zusammenhang auch ein Druck angesehen, der geringfügig von dem Druck abweicht, bei dem die Beschichtung mit Wirkstoff durchgeführt wird, sofern der Ballondurchmesser weitgehend übereinstimmt. Alternativ zur Benetzung durch Eintauchen kann die Benetzung wiederum auch auf andere Weise erfolgen, bspw. durch Besprühen. Nach den einzelnen Beschichtungsschritten ist ein Trocknungsschritt sinnvoll. Bei flüchtigen Lösungsmitteln erfolgt die Trocknung unter Umständen unmittelbar, bei anderen Lösungsmitteln kann die Trocknung durch Versetzen des Ballons in Rotation oder durch einen Luftstrom unterstützt werden.

Grundsätzlich können auch bestimmte Beschichtungsschritte wiederholt werden. Beispielsweise ist es möglich, zur Erhöhung der Wirkstoffbeladung den Ballon mehrfach mit einer Wirkstofflösung in Kontakt zu bringen. Ggf. ist es auch möglich, in diesem Zusammenhang unterschiedliche Wirkstoffe aufzubringen.

Neben dem erfindungsgemäßen Verfahren betrifft die Erfindung auch einen Ballon mit einer Beschichtung, wie sie sich durch das beschriebene Verfahren erzielen lässt. Dieses zeichnet sich dadurch aus, dass bei starker Expansion des Ballons über die Expansion des Ballons hinaus, bei der die Beschichtung stattgefunden hat, starke Scherkräfte wirken, die ein Abplatzen der Beschichtung und damit eine Abgabe von der Ballonoberfläche an die Innenwandung des Blutgefäßes/des umliegenden Gewebes herbeiführen. Des Weiteren betrifft die Erfindung einen Ballonkatheter mit einem solchen Ballon. Die Ballonkatheter sind, je nach Dimensionierung, in unterschiedlichsten Bereichen des Blutgefäßsystems einsetzbar, nämlich insbesondere im koronaren, intrakraniellen und peripheren Bereich.

Der erfindungsgemäße Ballonkatheter weist typischerweise Lumen, vorzugsweise zumindest zwei Lumen auf, wobei ein Lumen der Fluidzufuhr und Druckbeaufschlagung dient und mit dem Balloninneren in Verbindung steht, während das andere Lumen der Aufnahme eines Führungsdrahts dient, der zunächst zum Zielort vorangeschoben wird, um anschließend den Ballonkatheter über den Führungsdraht an den Zielort zu bringen. In diesem Zusammenhang sind im Wesentlichen zwei unterschiedliche Systeme aus dem Stand der Technik bekannt, nämlich Over-The-Wire (OTW)- und Rapid Exchange (Rx)-Ballonkatheter. Der erfindungsgemäße Ballonkatheter kann sowohl als OTW- als auch als Rx-Ballonkatheter vorliegen. Während sich bei einem OTW-Katheter das Lumen für den Führungsdraht über die gesamte Länge des Katheters von proximal nach distal erstreckt, verfügt der Rx-Katheter über eine separate Zuführöffnung für den Führungsdraht (Rx-Port), an der der Führungsdraht deutlich distal des proximalen Endes des Katheters aus dem Katheter austritt. Entsprechend laufen die Lumen für die Fluidzufuhr und den Führungsdraht im Falle eines OTW-Ballonkatheters parallel oder konzentrisch zueinander vom proximalen Ende des Katheters bis zum Ballon, während dies bei einem Rx-Katheter nur zwischen Rx-Port und Ballon der Fall ist. Der Abschnitt zwischen Rx-Port und proximalem Ende hingegen weist nur ein Lumen für die Fluidzufuhr auf. Typischerweise verlaufen die Lumen in den Bereichen, in denen der Katheter zwei Lumen aufweist, parallel zueinander. Möglich ist auch ein konzentrischer Verlauf, bei dem das engere innere Lumen für den Führungsdraht durch das weitere äußere Lumen für die Fluidzufuhr verläuft.

Am proximalen Ende des Ballonkatheters ist zumeist ein sog. Katheterhub vorgesehen, d. h. ein Anschlussstück für die Vorrichtung zur Fluidzufuhr und Druckbeaufschlagung. Die Verbindung kann z. B. eine herkömmliche Luer- bzw. Luer-Lock-Verbindung sein. Unter proximal wird in Richtung Körperäußeres, d. h. zum behandelnden Arzt hin verstanden, unter distal die entgegengesetzte Richtung, d. h. in Richtung des zu behandelnden Blutgefäßes. Die Einfuhr des Ballonkatheters in den menschlichen Körper erfolgt zumeist in der Leistengegend über die Arteria femoralis.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung handeln.

### Beispiel

Ein Ballon aus einem elastischen Polyurethan wird von innen mit einem Druck von 3 bar beaufschlagt. Bei diesem Druck weist der Ballon einen Durchmesser von 4,5 mm auf. In diesem Zustand wird der Ballon in eine Lösung von Paclitaxel in Methylenchlorid eingetaucht und langsam wieder herausgezogen. Die Konzentration des Paclitaxel beträgt 200 mg/ml. Die Beschichtung erfolgt bei Raumtemperatur. Anschließend wird der Druck abgebaut, woraufhin der Ballon sich eng zusammenfaltet.

Der Ballon ist Teil eines Ballonkatheters, der in den menschlichen Körper eingeführt und durch das Blutgefäßsystem an den Zielort vorgeschoben wird. Dort erfolgt eine Druckbeaufschlagung mit 6 bar. Bei diesem Druck beträgt der Durchmesser 6 mm, d. h. Durchmesser und Oberfläche haben sich gegenüber dem Beschichtungsschritt um 33 % vergrößert. Dies bewirkt die Entstehung starker Scherkräfte und ein Absprengen der Wirkstoffschicht, die an die Gefäßinnenwandung abgegeben wird.

**Gemäß vorteilhafter Ausführungsformen betrifft die Erfindung Verfahren, Ballons und Ballonkatheter gemäß folgender Positionen:**

1. Verfahren zur Beschichtung der Oberfläche des Ballons eines Ballonkatheters mit einem Wirkstoff, wobei der Ballon aus einem elastischen Material gefertigt und durch Druckbeaufschlagung mit einem Fluid expandierbar ist, wobei der Ballon dafür vorgesehen ist, an einem Zielort expandiert zu werden, wobei die Beschichtung der Oberfläche des Ballons bei einem Druck erfolgt, der unterhalb des Drucks liegt, der für die Expansion des Ballons am Zielort aufgewandt wird.

2. Verfahren nach Position 1, wobei der Druck, bei dem die Beschichtung erfolgt, mindestens 20 %, vorzugsweise mindestens 30 % unterhalb des Drucks liegt, der für die Expansion des Ballons am Zielort aufgewandt wird.

3. Verfahren nach Position 2, wobei der Druck, bei dem die Beschichtung erfolgt, maximal 50 % des Drucks beträgt, der für die Expansion des Ballons am Zielort aufgewandt wird.

4. Verfahren nach einer der Positionen 1 bis 3, wobei das elastische Material ein Polyurethan, ein Polyolefincopolymer, ein Polyethylen oder ein Silikon umfasst.

5. Verfahren nach einer der Positionen 1 bis 3, wobei das elastische Material ein thermoplastisches Elastomer, insbesondere ein Polyetherblockamid umfasst.

6. Verfahren nach einer der Positionen 1 bis 5, wobei die Zunahme des Durchmessers des Ballons bei Verdopplung des Drucks, ausgehend vom Nominaldruck, bei dem der Ballon seinen nominalen Durchmesser erreicht, mindestens 5 %, vorzugsweise mindestens 10 %, weiter vorzugsweise mindestens 20 % und besonders bevorzugt mindestens 30 % beträgt.

7. Verfahren nach einer der Positionen 1 bis 6, wobei der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Tacrolimus, hydrophobe Proteine, Heparin und/oder hormonartige oder zellproliferationsverändernde Substanzen.

8. Verfahren nach einer der Positionen 1 bis 7, wobei zumindest der mit dem Wirkstoff beschichtete Teil der Oberfläche des Ballons mit einer Wasser und/oder mindestens einen Alkohol enthaltenden Flüssigkeit benetzt wird.

9. Verfahren nach einer der Positionen 1 bis 8, wobei zumindest der mit dem Wirkstoff beschichtete Teil der Oberfläche des Ballons vor oder nach der Beschichtung mit dem Wirkstoff mit einem Polysaccharid beschichtet wird.

10. Verfahren nach Position 9, wobei die mittlere Molmasse des Polysaccharids 10.000 bis 100.000.000 Da beträgt.

11. Verfahren nach Position 10, wobei die mittlere Molmasse des Polysaccharids 20.000 bis 80.000 Da beträgt.

12. Verfahren nach einer der Positionen 9 bis 11, wobei das Polysaccharid ein Dextran ist.

13. Ballon eines Ballonkatheters, dessen Oberfläche zumindest teilweise eine Beschichtung mit einem Wirkstoff aufweist, erhältlich durch ein Verfahren nach einer der Positionen 1 bis 12.

14. Ballonkatheter umfassend einen Ballon nach Position 13.

**Patentansprüche**

1. Verfahren zur Beschichtung der Oberfläche des Ballons eines Ballonkatheters mit einem Wirkstoff, wobei der Ballon aus einem elastischen Material gefertigt und durch Druckbeaufschlagung mit einem Fluid expandierbar ist, wobei der Ballon dafür vorgesehen ist, an einem Zielort expandiert zu werden, und die Beschichtung der Oberfläche des Ballons bei einem Druck erfolgt, der unterhalb des Drucks liegt, der für die Expansion des Ballons am Zielort aufgewandt wird, **dadurch gekennzeichnet, dass** zumindest der mit dem Wirkstoff beschichtete Teil der Oberfläche des Ballons mit einer Wasser und/oder mindestens einen Alkohol enthaltenden Flüssigkeit benetzt wird und die Oberfläche des Ballons bei der Expansion am Zielort um mindestens 10 % größer ist als während der Beschichtung.

2. Verfahren nach Anspruch 1, **dadurch gekennzeichnet, dass** der Druck, bei dem die Beschichtung erfolgt, mindestens 20 %, vorzugsweise mindestens 30 % unterhalb des Drucks liegt, der für die Expansion des Ballons am Zielort aufgewandt wird.

3. Verfahren nach Anspruch 2, **dadurch gekennzeichnet, dass** der Druck, bei dem die Beschichtung erfolgt, maximal 50 % des Drucks beträgt, der für die Expansion des Ballons am Zielort aufgewandt wird.

4. Verfahren nach einem der Ansprüche 1 bis 3, **dadurch gekennzeichnet, dass** das elastische Material ein Polyurethan, ein Polyolefincopolymer, ein Polyethylen oder ein Silikon umfasst.

5. Verfahren nach einem der Ansprüche 1 bis 3, **dadurch gekennzeichnet, dass** das elastische Material ein thermoplastisches Elastomer, insbesondere ein Polyetherblockamid umfasst.

6. Verfahren nach einem der Ansprüche 1 bis 5, **dadurch gekennzeichnet, dass** die Oberfläche des Ballons bei der Expansion am Zielort um mindestens 20 %, bevorzugt mindestens 30 %, weiter bevorzugt mindestens 40 % und sehr bevorzugt mindestens 50 % größer ist als während der Beschichtung.

7. Verfahren nach einem der Ansprüche 1 bis 6, **dadurch gekennzeichnet, dass** der verwendete Wirkstoff ausgewählt ist aus der Gruppe: Tretinoin, Orphanrezeptoragonisten, Elafinderivate, Corticosteroide, Steroidhormone, Paclitaxel, Rapamycin, Tacrolimus, hydrophobe Proteine, Heparin und/oder hormonartige oder zellproliferationsverändernde Substanzen.

8. Verfahren nach einem der Ansprüche 1 bis 7, **dadurch gekennzeichnet, dass** zumindest der mit dem Wirkstoff beschichtete Teil der Oberfläche des Ballons vor oder nach der Beschichtung mit dem Wirkstoff mit einem Polysaccharid beschichtet wird.

9. Verfahren nach Anspruch 8, **dadurch gekennzeichnet, dass** die mittlere Molmasse des Polysaccharids 10.000 bis 100.000.000 Da beträgt.

10. Verfahren nach Anspruch 9, **dadurch gekennzeichnet, dass** die mittlere Molmasse des Polysaccharids 20.000 bis 80.000 Da beträgt.

11. Verfahren nach einem der Ansprüche 8 bis 10, **dadurch gekennzeichnet, dass** das Polysaccharid ein Dextran ist.

12. Ballon eines Ballonkatheters, dessen Oberfläche zumindest teilweise eine Beschichtung mit einem Wirkstoff aufweist, erhältlich durch ein Verfahren nach einem der Ansprüche 1 bis 11.

13. Ballonkatheter umfassend einen Ballon nach Anspruch 12.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013178820 A1